Europäisches Patentamt

European Patent Office

Office européen des brevets

(19)

(11) Publication number: **0 304 311**
**A1**

(12)

# EUROPEAN PATENT APPLICATION

(21) Application number: 88307688.7

(22) Date of filing: 19.08.88

(51) Int. Cl.⁴: **C 12 N 11/08**
**A 61 K 37/547**

(30) Priority: 21.08.87 GB 8719825
06.10.87 GB 8723468

(43) Date of publication of application:
22.02.89 Bulletin 89/08

(84) Designated Contracting States:
AT BE CH DE ES FR GB GR IT LI LU NL SE

(71) Applicant: THE WELLCOME FOUNDATION LIMITED
183-193 Euston Road
London NW1 2BP (GB)

(72) Inventor: Berger Jr., Henry
610 Normandy Street
Cary North Carolina (US)

Pizzo, Salvatore Vincent
Box 442, Route 2 Cole Mill Road
Durham North Carolina 27705 (US)

(74) Representative: Stott, Michael John et al
The Wellcome Research Laboratories Group Patents &
Agreements Langley Court
Beckenham Kent BR3 3BS (GB)

The title of the invention has been amended (Guidelines for Examination in the EPO, A-III, 7.3).

(54) Complex of polyethylene glycol and tissue plasminogen activator.

(57) A complex of PEG and t-PA coupled together via a carbonyl group has an enhanced clearance time.

EP 0 304 311 A1

**Description**

## NOVEL COMPLEX

The present invention relates to a complex of polyethylene glycol and tissue plasminogen activator, to a process for its preparation, to pharmaceutical formulations containing it, and to its use in human and veterinary medicine.

There exists a dynamic equilibrium between the enzyme system capable of forming blood clots, the coagulation system, and the enzyme system capable of dissolving blood clots, the fibrinolytic system, which maintains an intact patent vascular bed. To limit loss of blood from injury, blood clots are formed in the injured vessel. After natural repair of the injury, the superfluous blood clots are dissolved through operation of the fibrinolytic system. Occasionally, blood clots form without traumatic injury and may lodge in major blood vessels resulting in a partial or even total obstruction to blood flow. When this occurs in the heart, lung or brain, the result may be a myocardial infarction, pulmonary embolism or stroke. These conditions combined are the leading cause of morbidity and mortality in the industrialised nations.

Blood clots consist of a fibrous network that is capable of dissolution by the proteolytic enzyme plasmin. The enzyme is derived from the inactive proenzyme, plasminogen, a component of blood plasma by the action of a plasminogen activator. There are two immunologically distinct mammalian plasminogen activators. Intrinsic plasminogen activator, also known as urokinase, is an enzyme produced by the kidney and can be isolated from urine. It can also be prepared from a number of tissue culture sources. Extrinsic plasminogen activator, also known as vascular plasminogen activator and as tissue plasminogen activator (t-PA), can be isolated from many tissue homogenates (notably human uterus), the vascular cell wall and from some cell cultures. In addition to these two kinds of plasminogen activator, there is also a bacterial product, streptokinase, prepared from beta-haemolytic streptococci. A major drawback with both urokinase and streptokinase is that they are active throughout the circulation and not just at the site of a blood clot. They can, for example, destroy other blood proteins, such as fibrinogen, prothrombin, factor V and factor VIII so reducing blood clotting ability and increasing the risk of haemorrhage. In contrast, the biological activity of t-PA is dependent on the presence of fibrin to which it binds and where it is activated. Maximum activity is thus developed only at the site of a blood clot, i.e. in the presence of the fibrin network to be dissolved, and this greatly avoids the risk of haemorrhage.

The clearance of t-PA from the circulation normally occurs through the liver and takes several minutes in the case of the two-chain form and even less in the case of the one-chain form. Whilst this is acceptable for intravascular infusions, it may be too short for a bolus injection as the protein would probably be cleared from the system before it had an opportunity to exert its full fibrinolytic effect. It would, therefore, be desirable to increase the clearance time of t-PA in some way without significantly impairing its ability to bind to fibrin and to dissolve blood clots.

It is known from US patent 4179337 that the clearance times of certain enzymes can be increased by complexing them with polyethylene glycol (PEG). It is also known from British patent specification 2110219 that polyalkylene glycol complexes of human plasminogen activators can be prepared and that these have a prolonged half life in the blood. However the specific disclosure of this specification relates to urokinase and there is no disclosure of reagents or conditions which will yield a complex of PEG and t-PA.

It has now been found that a novel complex of PEG and t-PA can be obtained which has a significantly increased clearance time compared with that of t-PA per se.

Accordingly, the present invention provides a complex of PEG and t-PA in which the PEG is coupled to the t-PA via a carbonyl group.

The structure of the complex in the region where the coupling between the molecules occurs is preferably of formula:

$ACH_2O-CO-NHB$

wherein $ACH_2O-$ and $-NHB$ are derivatives of PEG and t-PA respectively, $ACH_2OH$ representing PEG and $H_2NB$ representing t-PA. Thus the complexing preferably occurs through one of the terminal groups of the PEG and through one of the free amino groups of the lysine residues or the amino terminus of the t-PA.

The extent of coupling in terms of the number of molecules of PEG complexed with each molecule of t-PA may vary. It is however generally not desirable for the extent of complexing to be too great since this may lead to a decrease in the ability of the complex to activate plasminogan to plasmin. Optimally there are from 2 to 6 molecules of PEG for each molecule of t-PA.

The complex of the present invention has a clearance time which is at least several times longer than the clearance time for t-PA itself. It also has greater fibrinolytic activity than complexes in which the PEG and the t-PA are coupled together differently, for example via a chlorotriazinyl or succinyl group. The complex may be used to remove a blood clot and also to prevent formation of a clot in a patient where this is likely to occur, for example following recanalization of a blood vessel of a patient. Although the complex of the present invention may be used to remove or prevent formation of a clot in any blood vessel, it is particularly useful in removing or preventing the formation of a coronary clot.

The t-PA which is used as starting material to form the complex of the present invention may be any bioactive protein substantially corresponding to mammalian, and especially human, t-PA and includes forms with and without glycosylation. It may be one- or two-chain t-PA, or a mixture thereof, as described in EP-A-112 122, and, in the case of fully glycosylated human t-PA, has an apparent molecular weight on polyacrylamide gel of about 70,000 and an isoelectric point of between 7.5 and 8.0. Preferably the t-PA has a specific activity of about 500,000 IU/mg (international Units/mg, the International Unit being a unit of activity as defined by WHO, National Institute for Biological Standards and Control, Holly Hill, Hampstead, London NW3 6RB, U.K.)

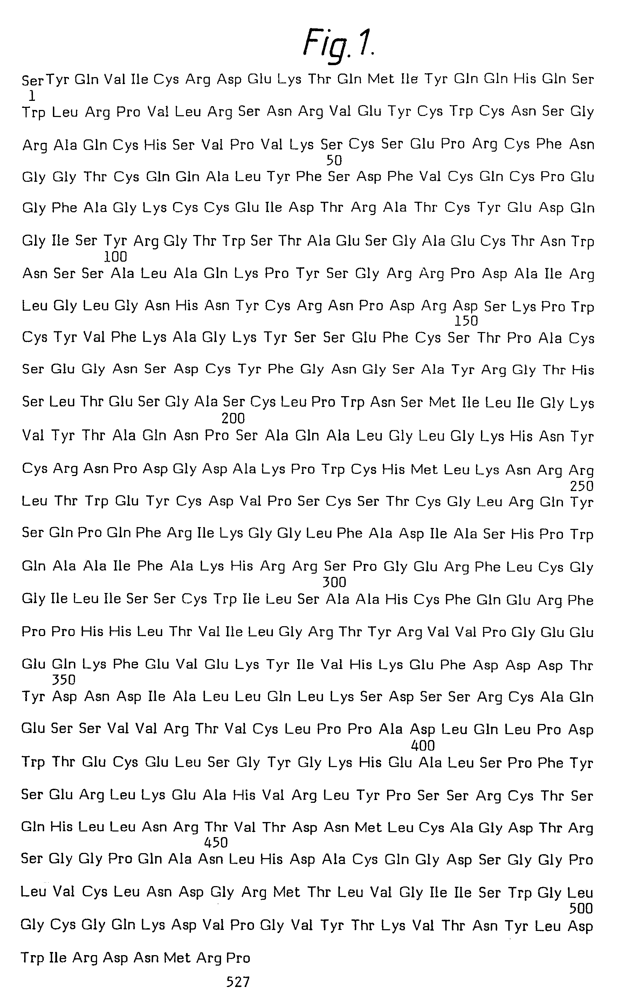

The amino acid sequence of t-PA preferably substantially corresponds to that set forth in Figure 1. The sequence is thus identical to that in Figure 1 or contains one or more amino acid deletions, substitutions, insertions, inversions or additions of allelic origin or otherwise, the resulting sequence having at least 80% and preferably 90%, homology with the sequence in Figure 1 and retaining essentially the same biological and immunological properties of the protein. In particular, the sequence is identical to that in Figure 1 or has the same sequence but with the amino acid in the 245th position from the serine N-terminus being valine instead of methionine, either sequence optionally being without any of the first three amino acids or optionally having an additional polypeptide N-terminal presequence of Gly-Ala-Arg.

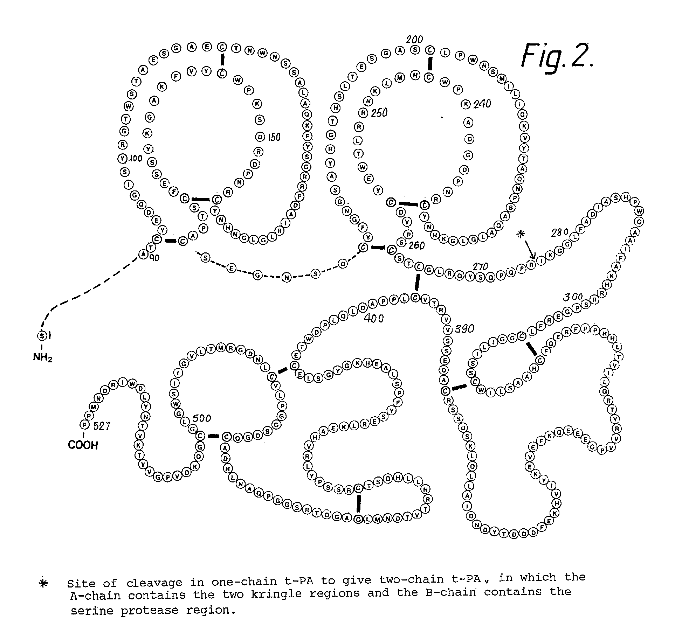

The amino acid sequence set forth in Figure 1 has thirty-five cysteine residues and thus the potential for forming seventeen disulphide bridges. Based on analogy with other proteins whose structure has been determined in more detail, the postulated structure for the sequence (arising from disulphide bond formation) between the amino acid in the 90th position and the proline C-terminus is set forth in Figure 2. The structure of the N-terminal region is less certain although some proposals have been put forward (Progress in Fibrinolysis, 1983, 6, 269-273; and Proc. Natl. Acad. Sci., 1984, 81, 5355-5359). The most important features of the structure of t-PA are the two kringle regions (between the 92nd and the 173rd amino acids and between the 180th and 261st amino acids), which are responsible for the binding of the protein to fibrin, and the serine protease region, which comprises the major part of the B-chain and which is responsible for the activation of plasminogen. The amino acids of special significance in serine proteases are the catalytic triad, His/Asp/Ser. In t-PA these occur at the 322nd, the 371st and the 463rd positions. The disulphide bridge between the 264th and 395th cysteine amino acid residues is also important in that it holds together the A- and the B-chains in the two-chain form of t-PA.

In Figures 1 and 2, the conventional one and three letter codes have been employed for the amino acid residue as follows:

| Asp | D | Aspartic acid |
| Thr | T | Threonine |
| Ser | S | Serine |
| Glu | E | Glutamic acid |
| Pro | P | Proline |
| Gly | G | Glycine |
| Ala | A | Alanine |
| Ile | I | Isoleucine |
| Leu | L | Leucine |
| Tyr | Y | Tyrosine |
| Phe | F | Phenylalanine |
| His | H | Histidine |
| Cys | C | Cysteine |
| Val | V | Valine |
| Lys | K | Lysine |
| Arg | R | Arginine |
| Trp | W | Tryptophan |
| Gln | Q | Glutamine |
| Met | M | Methionine |
| Asn | N | Asparagine |

The t-PA may be obtained by any of the procedures described or known in the art. For example, it may be obtained from a normal or neoplastic cell line of the kind described in Biochimica et Biophysica Acta, 1979, 580 140-153; EP-A-41 766 or EP-A-113 319. It is preferred, however, that t-PA is obtained from a cultured transformed or transfected cell line derived using recombinant DNA technology as described in, for example, EP-A-93 619; EP-A-117 059; EP-A-117 060; EP-A-173 552; EP-A-174 835; EP-A-178 105; EP-A-225 177; EP-A-225 286; WO 86/01538; WO 86/05514; or WO 86/05807. It is particularly preferred that Chinese hamster ovary (CHO) cells are used for the production of t-PA and are derived in the manner as described in Molecular and Cellular Biology, 1985, 5(7), 1750-1759. In this way, the cloned gene is cotransfected with the gene encoding dihydrofolate reductase (dhfr) into dhfr CHO cells. Transformants expressing dhfr are selected on media lacking nucleosides and are exposed to increasing concentrations of methotrexate. The dhfr and t-PA genes are thus coamplified leading to a stable cell line capable of expressing high levels of t-PA.

The t-PA is, preferably, purified using any of the procedures described or known in the art, such as the procedures described in Biochimica et Biophysica Acta, 1979, 580, 140-153; J. Biol. Chem., 1979, 254(6), 1998-2003; ibid, 1981, 256(13), 7035-7041; Eur. J. Biochem.,1983, 132, 681-686; EP-A-41 766; EP-A-113 319; or GB-A-2 122 219.

The PEG which is used as starting material to form the complex of the present invention is generally of formula

$$ROCH_2(CH_2OCH_2)_nCH_2OH$$

wherein n is from 5 to 500 and R is hydrogen or a substituent which prevents cross-linking between the PEG and the t-PA and which does not interfere with the function of the PEG in the complex of the present invention. It is preferred that there is univalent coupling between the PEG and the t-PA and that therefore R is not hydrogen In particular, R is $C_{1-4}$ alkyl, such as methyl.

The PEG will generally have a molecular weight in the range from 200 to 20,000, preferably 500 to 10,000. As the molecular weight of the PEG increases, the specific activity of the complex of the present invention tends to decrease whereas its clearance time increases. Optimally, the PEG has a molecular weight of about 5000. Suitable materials are available commercially, for example monomethoxy-polyethylene glycol of nominal molecular weight 5000 available from Polysciences Inc., Warrington, Pennsylvania, U.S.A.

It has also been found that the complex of the present invention can be prepared by carrying out the coupling reaction in the presence of an alkali metal thiocyanate.

Accordingly, the present invention also provides a process for preparing a complex of PEG and t-PA, as herein defined, which comprises coupling an activated PEG of formula:

ACH$_2$O-CO-X

wherein ACH$_2$O is as herein defined and X is an activating group, with t-PA in the presence of an alkali metal thiocyanate.

The activating group renders the PEG amenable to coupling with t-PA whilst forming no part of the final complex. A preferred example is imidazolyl.

Preferably, the alkali metal thiocyanate is potassium thiocyanate and is used at a concentration from 1 to 2M.

The coupling of activated PEG to t-PA is generally carried out under relatively mild conditions so as to minimise any loss of enzyme activity of t-PA. For example, it may be carried out in an aqueous buffer at pH about 8.5 containing 1M potassium thiocyanate for a period of about two days. On completion of the reaction, the complex is preferably separated from unreacted reagents as soon as possible using for example dialysis or ion exchange chromatography.

The reaction conditions are generally chosen to achieve complexing of a substantial proportion of the molecules of t-PA, preferably greater than 50%, with at least one molecule of activated PEG. The reaction conditions may also be adjusted to vary the extent of complexing in terms of the number of molecules of PEG complexed with each molecule of t-PA.

The activated PEG may be prepared by methods known in the art. In the preferred case where the activating group is imidazolyl, the activated PEG may be obtained by reacting PEG with 1,1-carbonyldiimidazole (CD1). Suitable conditions for this reaction are described in Analytical Biochemistry, 1983, 131, 25-33.

The complex of the present invention is preferably employed in the form of a pharmaceutical formulation. Accordingly the present invention provides a pharmaceutical formulation, which comprises a complex as herein defined and a pharmaceutically acceptable carrier.

Generally the complex of the present invention will be administered by the intravascular route, whether by infusion or by bolus injection, and thus a parenteral formulation is required. It is preferred to present a lyophilised formulation to the physician or veterinarian because of the significant transportation and storage advantages that it affords. The physician or veterinarian may then reconstitute the lyophilised formulation in an appropriate amount of solvent as and when required.

Parenteral and lyophilised pharmaceutical formulations containing t-PA are known in the art. Examples of such art include EP-A-41 766; EP-A-93 619; EP-A-112 122; EP-A-113 319; EP-A-123 304; EP-A-143 081; EP-A-156 169; EP-A-211 592; EP-A-217 379; EP-A-218 112; WO 86/01104; Japanese patent publication 57-120523 (Application 56-6936) and Japanese patent publication 58-65218 (Application 56-163145). Additional examples include GB-A-2 176 702 and GB-A-2 176 703. Parenteral and lyophilised formulations according to the present invention may be prepared in a similar manner to the preparation of the formulations known for t-PA per se. As well as acidic pH, the complex can be formulated at neutral pH to achieve solutions of at least 1mg of complex per ml.

Intravascular infusions are normally carried out with the parenteral solution contained within an infusion bag or bottle or within an electrically operated infusion syringe. The solution may be delivered from the infusion bag or bottle to the patient by gravity feed or by the use of an infusion pump. The use of gravity feed infusion systems dose not afford sufficient control over the rate of administration of the parenteral solution and, therefore, the use of an infusion pump is preferred especially with solutions containing relatively high concentrations of active ingredients. More preferred, however, is the use of an electrically operated infusion syringe which offers even greater control over the rate of administration.

The present invention also provides a method for removing or inhibiting the formation of a blood clot in a mammal, which comprises administering to the mammal an effective amount of a complex of PEG and t-PA. In the alternative, the present invention provides a complex of PEG and t-PA for use in human or veterinary medicine especially for use in removing or inhibiting the formation of a blood clot in a mammal.

An effective amount of the complex of PEG and t-PA to remove or inhibit the formation of a blood clot in a mammal will of course depend upon a number of factors including, for example, the age and weight of the mammal, the precise condition requiring treatment and its severity, the route of administration, the particular complex of PEG and t-PA employed, and will ultimately be at the discretion of the attendant physician or veterinarian. It is likely, however, than an effective amount will be from 0.1 mg/kg to 5 mg/kg depending on the specific activity and half life of the complex.

Thus, for a 70kg adult human being, an effective amount will generally be from 7 to 350 mg

The following example is provided in illustration of the present invention and should not be construed in any way as constituting a limitation thereof.

Example

### i) Activation of Monomethoxypolyethylene glycol

Monomethoxypolyethylene glycol of nominal molecular weight 5000 daltons (Polysciences Inc., Warrington, Pennsylvania, U.S.A. referred to hereinafter as "PEG") was activated with 1,1-carbonyldiimidazole (CDI) by the method described in Analytical Biochemistry, 131, 25-33 (1983). PEG (12g) was dissolved in 1,4-dioxane (50ml) at 37°C. When dissolution was complete CDI (4.055g) was added and the reaction mixture stirred for 2 hours at 37°C. The resulting activated PEG was dialysed extensively against water at 4°C in tubing having a molecular weight cut off of 2000 daltons. The activated PEG was then lyophilized and stored at -20°C.

### ii) Formation of PEG-t-PA Complex

t-PA (154mg) in the form of a partially purified preparation having a specific activity of $2.3 \times 10^5$ IU/mg was reacted for 45 hours at 4°C with activated PEG (0.44g) in 10ml sodium borate buffer (pH 8.5) containing 1M potassium thiocyanate. A small precipitate formed which was removed by centrifugation. The PEG-t-PA preparation was then dialysed overnight against 0.15M sodium chloride adjusted to pH 3. The dialysed preparation was diluted 4 fold with 0.05M sodium 2-(N-morpholino)ethanesulphonate (MES) buffer (pH 6.0) containing 1M urea and 0.1% Tween 80 (buffer A).

The PEG-t-PA was collected on a 1.5cm x 3cm phospho-cellulose column equilibriated in buffer A. After washing with several column volumes of buffer A, the PEG-t-PA was eluted with 0.05M MES buffer (pH 6.0) containing 0.2M sodium chloride, 1M urea and 0.1% Tween 80. Unreacted t-PA was next eluted using a 0.1M Tris,HCl buffer (pH 8.0) containing 1M sodium chloride, 4M urea and 0.1% Tween 80. These fractions were analysed by sodium dodecylsulphate polyacrylamide gel electrophoresis to verify the above. About 67% of the initial t-PA was derivatized with PEG and the PEG-t-PA complex had a specific activity of $8 \times 10^4$ IU/mg as determined by a radioactive fibrin plate assay (Thrombosis and Haemostasis 47, 166-172 (1982)).

### iii) Formulation of PEG-t-PA Complex

The PEG-t-PA complex was dialyzed against phosphate-buffered saline of the following composition: 8.0 g/l NaCl; 0.2 g/l KCl. l.15 g/l $Na_2HPO_4$; 0.2 g/l $KH_2PO_4$. The pH of the resulting solution was 7.2-7.4. The solution was sterilized by filtration through a 0.2 μ filter.

### iv) Assessment of Thrombolytic Efficacy

Male beagles were anesthetised with pentobarbital sodium, intubated and ventilated with room air via a Harvard respirator. Catheters for drug administration and arterial pressure measurement were implanted in the left jugular vein and left carotid artery respectively. A thoracotomy was performed at the 4th intercostal space, the heart suspended in a pericardial cradle, and the left anterior descending artery (LAD) isolated just below the first major diagonal branch. An electromagnetic flow probe was placed on the LAD. Snares were placed 1cm apart proximal to the flow probe. One NIH unit of thrombin was injected into the isolated segment via a side branch catheter which was thereafter ligated. After 30 minutes the snares were removed and the presence of a thrombotic occlusion verified by the persistence of cyanosis in the areas of the heart supplied by the LAD and the absence of flow monitored by the flow probe.

Formulated PEG-t-PA was administered by bolus injection at a dose of $5 \times 10^4$ IU/kg body weight and resulted in lysis of the thrombus and restoration of flow in 13 minutes. In contrast, bolus injection of native t-PA in sterile, physiological saline adjusted to pH 3 at a dose of $3.5 \times 10^5$ IU/kg body weight did not result in thrombolysis over the 60 minute period of observation. This same dose of native t-PA, however, administered by constant intravenous infusion over a 60 minute period resulted in thrombolysis and restoration of flow in 16 minutes. The elimination half-life of PEG-t-PA was 16 minutes versus 2-3 minutes for native t-PA.

## Claims

1. A complex of formula:

$$ACH_2O\text{-}CO\text{-}NHB$$

wherein $ACH_2O$- and -NHB are derivatives of PEG and t-PA respectively, $ACH_2OH$ representing PEG and $H_2NB$ representing t-PA.

2. A complex according to claim 1, wherein there are from 2 to 6 molecules of PEG for each molecule of t-PA.

3. A complex according to either of the preceding claims, wherein, prior to coupling t-PA has the amino acid sequence set forth in Figure 1 or has the same sequence but with the amino acid in the 245th position from the serine N-terminus being valine instead of methionine.

4. A complex according to any one of the preceding claims, wherein, prior to coupling, PEG is monomethoxypolyethylene glycol.

5. A process for preparing a complex, as defined in any one of the preceding claims, which comprises coupling an activated PEG of formula:

$$ACH_2O\text{-}CO\text{-}X$$

wherein $ACH_2O$- is as defined in claim 1 and X is an activating group, with t-PA in the presence of an alkali metal thiocyanate.

6. A process according to claim 5, wherein the activating group is imidazolyl.

7. A pharmaceutical formulation, which comprises a complex, as defined in any one of claims 1 to 4, and a pharmaceutically accept-

EP 0 304 311 A1

# Fig.1.

SerTyr Gln Val Ile Cys Arg Asp Glu Lys Thr Gln Met Ile Tyr Gln Gln His Gln Ser

Trp Leu Arg Pro Val Leu Arg Ser Asn Arg Val Glu Tyr Cys Trp Cys Asn Ser Gly

Arg Ala Gln Cys His Ser Val Pro Val Lys Ser Cys Ser Glu Pro Arg Cys Phe Asn

Gly Gly Thr Cys Gln Gln Ala Leu Tyr Phe Ser Asp Phe Val Cys Gln Cys Pro Glu

Gly Phe Ala Gly Lys Cys Cys Glu Ile Asp Thr Arg Ala Thr Cys Tyr Glu Asp Gln

Gly Ile Ser Tyr Arg Gly Thr Trp Ser Thr Ala Glu Ser Gly Ala Glu Cys Thr Asn Trp

Asn Ser Ser Ala Leu Ala Gln Lys Pro Tyr Ser Gly Arg Arg Pro Asp Ala Ile Arg

Leu Gly Leu Gly Asn His Asn Tyr Cys Arg Asn Pro Asp Arg Asp Ser Lys Pro Trp

Cys Tyr Val Phe Lys Ala Gly Lys Tyr Ser Ser Glu Phe Cys Ser Thr Pro Ala Cys

Ser Glu Gly Asn Ser Asp Cys Tyr Phe Gly Asn Gly Ser Ala Tyr Arg Gly Thr His

Ser Leu Thr Glu Ser Gly Ala Ser Cys Leu Pro Trp Asn Ser Met Ile Leu Ile Gly Lys

Val Tyr Thr Ala Gln Asn Pro Ser Ala Gln Ala Leu Gly Leu Gly Lys His Asn Tyr

Cys Arg Asn Pro Asp Gly Asp Ala Lys Pro Trp Cys His Met Leu Lys Asn Arg Arg

Leu Thr Trp Glu Tyr Cys Asp Val Pro Ser Cys Ser Thr Cys Gly Leu Arg Gln Tyr

Ser Gln Pro Gln Phe Arg Ile Lys Gly Gly Leu Phe Ala Asp Ile Ala Ser His Pro Trp

Gln Ala Ala Ile Phe Ala Lys His Arg Arg Ser Pro Gly Glu Arg Phe Leu Cys Gly

Gly Ile Leu Ile Ser Ser Cys Trp Ile Leu Ser Ala Ala His Cys Phe Gln Glu Arg Phe

Pro Pro His His Leu Thr Val Ile Leu Gly Arg Thr Tyr Arg Val Val Pro Gly Glu Glu

Glu Gln Lys Phe Glu Val Glu Lys Tyr Ile Val His Lys Glu Phe Asp Asp Asp Thr

Tyr Asp Asn Asp Ile Ala Leu Leu Gln Leu Lys Ser Asp Ser Ser Arg Cys Ala Gln

Glu Ser Ser Val Val Arg Thr Val Cys Leu Pro Pro Ala Asp Leu Gln Leu Pro Asp

Trp Thr Glu Cys Glu Leu Ser Gly Tyr Gly Lys His Glu Ala Leu Ser Pro Phe Tyr

Ser Glu Arg Leu Lys Glu Ala His Val Arg Leu Tyr Pro Ser Ser Arg Cys Thr Ser

Gln His Leu Leu Asn Arg Thr Val Thr Asp Asn Met Leu Cys Ala Gly Asp Thr Arg

Ser Gly Gly Pro Gln Ala Asn Leu His Asp Ala Cys Gln Gly Asp Ser Gly Gly Pro

Leu Val Cys Leu Asn Asp Gly Arg Met Thr Leu Val Gly Ile Ile Ser Trp Gly Leu

Gly Cys Gly Gln Lys Asp Val Pro Gly Val Tyr Thr Lys Val Thr Asn Tyr Leu Asp

Trp Ile Arg Asp Asn Met Arg Pro

527

Fig.2.

* Site of cleavage in one-chain t-PA to give two-chain t-PA, in which the A-chain contains the two kringle regions and the B-chain contains the serine protease region.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | BLOOD, vol. 71, no. 6, June 1988, pages 1641-1647, Grune & Stratton, Inc.; H. BERGER, Jr.: "Preparation of polyethylene glycol-tissue plasminogen activator adducts that retain functional activity: characteristics and behavior in three animal species" * Whole article * | 1-9 | C 12 N 11/08 A 61 K 37/547 |
| Y | EP-A-0 183 503 (BEECHAM GROUP) * Claims 5,6 * | 1-9 | |
| Y | EP-A-0 154 432 (NIHON CHEMICAL RESEARCH K.K.) * Claims * | 1-9 | |
| A | FR-A-2 515 684 (NIPPON CHEMIPHAR) | | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 11-10-1988 | TURMO Y BLANCO C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)